(19) Europäisches Patentamt
European Patent Office
Office européen des brevets

(11) EP 4 806 923 A1

(12) EUROPEAN PATENT APPLICATION
published in accordance with Art. 153(4) EPC

(43) Date of publication:
**16.09.2026 Bulletin 2026/38**

(21) Application number: **24889111.1**

(22) Date of filing: **06.11.2024**

(51) International Patent Classification (IPC):
***C07K 17/06*** *(2006.01)* ***C12N 5/074*** *(2010.01)*
***A61K 35/17*** *(2025.01)*

(52) Cooperative Patent Classification (CPC):
**A61K 35/17; C07K 17/06; C12N 5/06**

(86) International application number:
**PCT/KR2024/017375**

(87) International publication number:
**WO 2025/100924 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.11.2023 KR 20230152073**
**14.02.2024 KR 20240021286**
**04.11.2024 KR 20240154399**

(71) Applicant: **LG Chem, Ltd.**
**Seoul 07336 (KR)**

(72) Inventors:
- **SONG, Seuk Young**
**Daejeon 34122 (KR)**
- **KIM, Jee Seon**
**Daejeon 34122 (KR)**
- **SHIN, Jung Youn**
**Daejeon 34122 (KR)**
- **OH, Seungjun**
**Daejeon 34122 (KR)**
- **KIM, Minchae**
**Daejeon 34122 (KR)**
- **KIM, Youngkyun**
**Daejeon 34122 (KR)**
- **KIM, Yeji**
**Daejeon 34122 (KR)**

(74) Representative: **Plasseraud IP**
**104 Rue de Richelieu**
**CS92104**
**75080 Paris Cedex 02 (FR)**

# (54) PARTICLE COMPLEX, CELL CULTURE COMPOSITION USING SAME, AND METHOD FOR PRODUCING CELL THERAPEUTIC AGENT

(57) The present disclosure relates to a particle composite comprising: a spherical particle; and a protein immobilized on the particle surface, and a cell culture composition and a preparation method for a cell therapeutic agent using the same.

[FIG. 1]

X 100
5.0kV
SEI
100µm
LM
WD 8.0mm

EP 4 806 923 A1

## Description

## [TECHNICAL FIELD]

Cross-Reference to Related Application(s)

This application claims priority to and the benefit of Korean Patent Application No. 10-2023-0152073, filed on November 6, 2023, Korean Patent Application No. 10-2024-0021286, filed on February 14, 2024, and Korean Patent Application No. 10-2024-0154399, filed on November 4, 2024, with the Korean Intellectual Property Office, the disclosures of which are incorporated herein by reference in their entirety.

The present disclosure relates to a particle composite, and a cell culture composition and a preparation method for a cell therapeutic agent using the same.

## [BACKGROUND]

As the application fields of biopharmaceuticals and regenerative medicine are expanded, the demand for mass cell culture technologies capable of efficiently producing cells, tissues, microorganisms, etc. is increasing.

Meanwhile, existing anticancer cell therapeutic agents use T cells (autologous cells) derived from each patient, and thus require a long time and is expensive to grow the cells, and general cancer patients are in a state where the function of immune cells is lowered. Therefore, they have drawbacks that it is not possible to guarantee the quality that can be used for treatment.

To solve this problem, attempts have been made to differentiate and use stem cells such as allogeneic induced pluripotent stem cells (iPSCs). This differentiation requires stimulation using proteins, but when this is applied to a flat-bed (2D), there are limitations to mass production and automation.

Therefore, along with the 3D conversion of the process of utilizing particles, there is a need to develop technologies that can completely separate particles from cells after cultivation.

## [DETAILED DESCRIPTION OF THE INVENTION]

## [Technical Problem]

The present disclosure relates to a technology for providing a particle composite that can covalently immobilize a protein required for cell differentiation to ensure the stability of a differentiation platform, and can be completely separated after cell culture to ensure the stability of a cell therapeutic agent.

The present disclosure also relates to a cell culture composition using the particle composite.

In addition, the present disclosure relates to a preparation method for a cell therapeutic agent using the particle composite.

## [Technical Solution]

In order to achieve the above objects, provided herein is a particle composite comprising: a spherical particle; and a protein immobilized on the particle surface.

Also provided herein is a cell culture composition comprising a cell and the particle composite.

Further provided is a preparation method for a cell therapeutic agent, the method comprising the steps of: culturing the cell culture composition; and removing the particle composite from the culture result.

A particle composite, and a cell culture composition and a preparation method for a cell therapeutic agent using the same according to specific embodiments of the present disclosure will be described in more detail.

Unless explicitly stated herein, the technical terms used herein are for the purpose of describing specific embodiments only and is not intended to limit the scope of the invention.

As used herein, the singular forms "a," "an" and "the" are intended to include the plural forms as well, unless the context clearly indicates otherwise.

It should be understood that the terms "comprise," "include", "have", etc. are used herein to specify the presence of stated features, regions, integers, steps, operations, elements, and/or components, but do not preclude the presence or addition of one or more other features, regions, integers, steps, operations, elements, components, and/or groups thereof.

As used herein, the term "substituted" means that other functional groups instead of a hydrogen atom in the compound are bonded, and a position to be substituted is not limited as long as the position is a position at which the hydrogen atom is substituted, that is, a position at which the substituent can be substituted, and when two or more are substituted, the two or more substituents may be the same as or different from each other.

As used herein, the term "substituted or unsubstituted" means being unsubstituted or substituted with one or more substituents selected from the group consisting of deuterium; a halogen group; a cyano group; a nitro group; a hydroxyl group; a carbonyl group; an ester group; an imide group; an amide group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; or a heterocyclic group containing at least one of N, O, and S atoms, or being unsubstituted or substituted with a substituent to which two or more substituents are linked among the substituents exemplified above. For example, "the substituent to which two or more substituents are linked" can be a biphenyl group. That is, the biphenyl group can also be an aryl group, and can be interpreted as a substituent to which two phenyl groups are linked.

As used herein, the notation , or means a bond linked to another substituent group, and a direct bond means the case where no other atoms exist in the parts represented as L.

As used herein, the alkyl group is a monovalent functional group derived from an alkane, and may be a straight-chain or a branched-chain. The number of carbon atoms of the straight chain alkyl group is not particularly limited, but is preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, a (meth)acrylate includes both acrylate and methacrylate.

As used herein, the term "dispersed phase composition" means a composition that can form a dispersed phase (or liquid droplet) after being mixed with a continuous phase composition.

As used herein, the term "continuous phase composition" means a composition that can form a continuous phase after being mixed with a dispersed phase composition.

Unless otherwise specifically defined or explained herein, the temperature at which the preparation process is performed (or each preparation step) or the temperature at which the numerical features of the prepared particles are calculated or measured may be room temperature. Specifically, as used herein, the "room temperature" refers to a temperature in a state where the temperature is not particularly increased or decreased, for example, a temperature in the range of 15°C to 30°C.

Below, the present disclosure will be described in more detail.

### 1. Particle composite

According to an embodiment of the present disclosure, there is provided a particle composite comprising: a spherical particle; and a protein immobilized on the particle surface.

The present inventors have found through experiments that in the case of the particle composite of an embodiment, a protein can be immobilized on the spherical particle surface by a chemical covalent bond or a physical bond such as adsorption to ensure the structural stability of the particle composite, and the particle composite can be completely separated from the cell culture medium using a cell strainer to ensure the stability of the cell therapeutic agent, and completed the present disclosure.

Specifically, the particle composite of an embodiment may include particles. The shape of the particles may be spherical. The spherical particle not only is excellent in durability and not damaged or destroyed by external impact, but also have a uniform particle surface and a large surface area, which can increase the protein immobilization efficiency.

Whether the particle has a spherical shape can be visually determined through SEM. The spherical particle may include both a true spherical particle corresponding to a theoretically perfect sphere, and a particle that are not a true sphere but have a shape close to a true sphere.

More specifically, the roundness of the spherical particle related to the present embodiment is typically 0.88 or more, or 0.90 or more, or 0.91 or more, or 0.92 or more, or 1 or less, or 0.98 or less, or 0.97 or less, or 0.88 to 1, or 0.90 to 1, or 0.91 to 1, or 0.92 to 1.

If the roundness of the spherical particle is within the above range, there is a tendency to suppress the deterioration of particle durability and protein immobilization performance. In addition, the roundness is defined by the following equation, and when the roundness is 1, it becomes a theoretically true sphere. Conversely, as the particle shape is farther from a true sphere, the value of the roundness is smaller.

Roundness = Perimeter of equivalent circle having the same area as projected particle shape / Actual perimeter of projected particle shape

Specifically, the method of measuring the roundness is not particularly limited, but as an example, it can be measured from a cross-sectional SEM image. Specifically, the roundness is calculated from the following equation using the particle area S [$\mu m^2$] and the perimeter L [$\mu$m] obtained from the cross-sectional SEM image.

$$\text{Roundness} = 4\pi S / (L)^2$$

The method of taking the particle boundary is not particularly limited, and may be performed automatically or manually using commercially available analysis software. It is preferably approximated to a polygon, and in this case, it is more preferable to approximate to a 15 or more angular shape. This is because, if the shape is less than 15 angular, there are cases where the background part may be treated as inside the particle when approximating the curved part.

The image of the particle cross section uses a reflection electron image acquired at an acceleration voltage of 5 kV using a SEM (scanning electron microscope). The method for preparing a sample for SEM observation to obtain a cross-sectional image of a particle is not particularly limited, and the particle cross section is cut to prepare a sample, and then a cross-sectional image of a particle is acquired using a SEM. In addition, the imaging magnification is usually 10 times or more, or 50 times or more, or 100 times or more, or 1000 times or less.

On the other hand, when a conventionally widely used culture plate is used instead of the spherical particle, there exists limitations in that it is difficult to control the amount and uniformity of the protein because the protein is immobilized using simple adsorption rather than covalent bonds. In addition, since it is difficult to mass-produce and automate the protein coated onto the plate, it is essential to immobilize proteins to the particles for mass culture.

Further, the particle may have a D50 diameter of more than 20 $\mu$m, or less than 180 $\mu$m, or a diameter of more than 20 $\mu$m and less than 180 $\mu$m, or 30 $\mu$m to 175 $\mu$m, or 50 $\mu$m to 160 $\mu$m, or 50 $\mu$m to 130 $\mu$m, or 50 $\mu$m to 80 $\mu$m, or 80 $\mu$m to 120 $\mu$m, or 120 $\mu$m to 160 $\mu$m. When the D50 diameter of the particle satisfies the above-mentioned range, cell differentiation and culture performance are excellent.

The diameter of the particle means the distance between the two points where the straight line passing through the center of gravity of the particle meets the outermost surface of the particle, and the D50 diameter of the particle means the size of the 50th particle when the total number of particles is converted to 100.

The method of measuring the D50 diameter of the particle is not particularly limited, and various well-known methods for measuring the D50 diameter of particles can be applied without limitation. However, as an example, it can be measured by analyzing the SEM (scanning electron microscopy) image.

If the D50 diameter of the particle increases excessively outside the above-mentioned range, the surface area per volume decreases, which reduces the interaction between cells and particles, and thus may cause a problem that cell differentiation efficiency is lowered.

On the other hand, if the D50 diameter of the particle decreases excessively outside the above-mentioned range, it is difficult to completely separate the particle composite from the cell culture medium using a cell strainer, which may cause problems in the safety of the final cell therapeutic agent.

The particle may include a single particle or a group of particles consisting of multiple single particles. The single particle means one particle, and the group of particles means a plurality of particle matrices in which two or more particles are mixed.

In this case, the single particle may include a protein immobilized on the surface. That is, it may include a protein immobilized on the surface of a single particle, which is one particle. In addition, it may include a protein immobilized on the surface of each single particle in a particle group in which two or more particles are mixed.

The particle may be a suspension-polymerized particle. The suspension polymerization particle means a particle obtained through a step of mixing a continuous phase composition and a dispersed phase composition and performing polymerization in a suspension state.

The dispersed phase composition means a composition that can form a dispersed phase (or liquid droplet) after being mixed with a continuous phase composition, and the continuous phase composition means a composition that can form a continuous phase after being mixed with a dispersed phase composition.

In an exemplary embodiment, the dispersed phase composition may include a polymerizable monomer.

In an embodiment of the present application, the polymerizable monomer is a monomer having an unsaturated bond between carbons, which may be a monomer further including an epoxy group, an amide group, a carboxyl group, an alkoxy group, a sulfonate group, a thiol group, an amine group, or a hydroxyl group. The above-mentioned group may be referred to as a hydrophilic group.

In an exemplary embodiment, the polymerizable monomer may be a (meth)acrylate-based monomer. In this case, the (meth)acrylate-based monomer having a hydrophilic group may include one or more of the above-mentioned

hydrophilic groups.

Although not particularly limited, examples of the (meth)acrylate-based monomer having an epoxy group as a hydrophilic group may include glycidyl(meth)acrylate, 4,5-epoxybutyl(meth)acrylate, 9,10-epoxy stearyl(meth)acrylate, or the like.

Although not particularly limited, examples of the (meth)acrylate-based monomer having an amide group as a hydrophilic group may include (meth)acrylamide or N-methylol(meth)acrylamide.

Although not particularly limited, examples of the (meth)acrylate-based monomers having a carboxyl group as a hydrophilic group may include acrylic acid, methacrylic acid, maleic acid, itaconic acid, or the like.

Although not particularly limited, the (meth)acrylate-based monomer having an alkoxy group as a hydrophilic group may have a methoxy group or an ethoxy group. For example, the methoxyethyl (meth)acrylate or the like may be used.

Although not particularly limited, the (meth)acrylate-based monomer having a hydroxyl group as a hydrophilic group may be, for example, hydroxyethyl(meth)acrylate, hydroxypropyl(meth)acrylate, or hydroxybutyl(meth)acrylate.

One or more of the monomers listed above may be used as a (meth)acrylate monomer having a hydrophilic group. In a specific embodiment of the present application, a monomer having an unsaturated bond between carbons and an epoxy group can be used as the polymerizable monomer.

In an exemplary embodiment, the dispersed phase composition may further include a crosslinking agent. The usable crosslinking agent is not particularly limited, but for example, a multifunctional (meth)acrylate such as ethylene glycol di(meth)acrylate, trimethylolpropane tri(meth)acrylate, pentaerythritol tri(meth)acrylate, dipentaerythritol hexa(meth)acrylate, dipentaerythritol hexamethacrylate, or trimethylpropane trimethacrylate may be used. Alternatively, one or more of the crosslinking agents listed above may be used in combination. Considering the formation of a hydrophilic surface of the particle, or the like, it may be preferable to use ethylene glycol di(meth)acrylate.

In an exemplary embodiment, the dispersed phase composition may contain the crosslinking agent in an amount in the range of 50 to 500 parts by weight based on 100 parts by weight of the polymerizable monomer. Specifically, the content of the crosslinking agent may be 100 parts by weight or more, 150 parts by weight or more, 200 parts by weight or more, 250 parts by weight or more, or 300 parts by weight or more. And, the upper limit thereof may be, for example, 450 parts by weight or less, 400 parts by weight or less, or 350 parts by weight or less. When the content of the crosslinking agent satisfies the above content, it is advantageous for stably forming an emulsion and ensuring particle strength and morphological characteristics through a desired level of crosslinking.

In an exemplary embodiment, the dispersed phase composition may further include an initiator. The type of the initiator is not particularly limited as long as it does not become an obstacle to securing of particle characteristics according to the preparation method of the present application. For example, an initiator such as an organic peroxide initiator or an azo initiator may be used. Specifically, compounds such as benzoyl peroxide, di-t-amyl peroxide, t-butyl peroxybenzoate, 2,5-dimethyl-2,5-di-(t-butylperoxy)hexane, 2,5-dimethyl-2,5-di-(t-butylperoxy)hexyne-3 or di-cumyl peroxide, and mixtures thereof may be used, but are not limited thereto.

The content of the initiator is not particularly limited. The initiator may be used in an appropriate amount within the range that does not become an obstacle to securing of the desired particle characteristics. For example, the dispersed phase composition may include the initiator in an amount of 0.1 part by weight or more, specifically 0.2 part by weight or more, 0.3 part by weight or more, 0.4 part by weight or more, 0.5 part by weight or more, 0.6 part by weight or more, 0.7 part by weight or more, 0.8 part by weight or more, 0.9 part by weight or more, or 1.0 part by weight or more, based on total 100 parts by weight of the polymerizable monomer and the crosslinking agent. And, the upper limit of the initiator content may be, for example, 5.0 parts by weight or less, specifically 4.5 parts by weight or less, 4.0 parts by weight or less, 3.5 parts by weight or less, 3.0 parts by weight or less, 2.5 parts by weight or less, 2.0 parts by weight or less, or 1.5 parts by weight or less.

In an exemplary embodiment, the continuous phase composition may include water and a polymeric surfactant.

Although not particularly limited, the water may be distilled water or deionized water.

In an exemplary embodiment, the continuous phase composition may be a mixture of water and a polymeric surfactant. That is, the continuous phase composition may be composed of only water and a polymeric surfactant.

In a specific embodiment of the present application, the polymeric surfactant may have a weight average molecular weight(Mw) within a predetermined range and/or a hydrolyzed degree within a predetermined range.

In an exemplary embodiment, the weight average molecular weight of the polymeric surfactant may be 60,000 or more. Specifically, the lower limit of the weight average molecular weight may be, for example, 65,000 or more, 70,000 or more, 80,000 or more, or 85,000 or more. And, the upper limit of the weight average molecular weight may be, for example, 190,000 or less, 185,000 or less, 180,000 or less, 175,000 or less, 170,000 or less, 165,000 or less, 160,000 or less, 155,000 or less, 150,000 or less, 145,000 or less, 140,000 or less, 135,000 or less, 130,000 or less, 125,000 or less, 124,000 or less. The weight average molecular weight may be measured using GPC.

In an exemplary embodiment, the polymer surfactant may have a hydrolyzed degree in the range of 80 to 99%. More specifically, the lower limit of the hydrolyzed degree may be, for example, 81% or more, 82% or more, 83% or more,

84% or more, 85% or more, or 87% or more, and the upper limit thereof may be, for example, 98% or less, 97% or less, 96% or less, 95% or less, 94% or less, 93% or less, 92% or less, 91% or less, 90% or less, or 89% or less. As a surfactant having the hydrolyzed degree, for example, polyvinyl alcohol may be used. The hydrolyzed degree may be measured using 1H-NMR.

Examples of the usable polymer surfactant include polyvinyl alcohol (PVA) or polyvinylpyrrolidone.

In an exemplary embodiment, the concentration of the polymer surfactant in the continuous phase composition may be 1.0% or more. For example, when a continuous phase composition that is a mixture of water and a polymer surfactant is used, the content of the polymer surfactant may be 1.0% or more by weight based on the total weight (100 wt.%) of the continuous phase composition. Specifically, the lower limit of the concentration of the polymer surfactant in the continuous phase composition may be 1.5% or more by weight or 2.0% or more by weight. And, the upper limit of the concentration of the polymer surfactant may be less than 4.0%, specifically, for example, 3.5% or less, 3.0% or less, or 2.5% or less. When a polymer surfactant satisfying the above concentration is used, uniform and stable liquid droplets can be formed when the continuous phase and the dispersed phase are mixed, which advantageous for obtaining the final particle shape desired in the present application.

In an exemplary embodiment, the mixture containing a dispersed phase and a continuous phase (or the mixture of a dispersed phase and a continuous phase) can satisfy a viscosity in the range of 1.0 to 2.5 cP. Within the viscosity range, collisions and breakages between liquid crystals or particles can be reduced, and the particle shape produced can be made more uniform. The viscosity can be measured according to the shear rate. The viscosity can be measured using a rotational viscometer, i.e., LVDV2T (Brookfield), in a shear rate range of 66 to 264 1/s and at room temperature (15 to 30°C).

In an exemplary embodiment, the mixture containing a dispersed phase and a continuous phase can satisfy a density in the range of 0.99 to 1.05 g/cm$^3$.

The suspension polymerization can be performed under conditions that do not become an obstacle to securing of particle characteristics according to the preparation method of the present application. For example, the suspension polymerization reaction can be performed at a temperature of 100°C or less, more specifically, at a temperature of 40°C to 95°C. And, during the polymerization reaction at the above temperature, stirring can be performed at a speed of 600 rpm to 1,000 rpm.

In an exemplary embodiment, the method can further include a washing step after the completion of the suspension polymerization reaction. Impurities unrelated to the particles, which are the suspension polymer, can be removed by washing. The washing method is not particularly limited, and a known washing method can be used. For example, the washing can be performed by adding the suspension polymer to a washing solution containing alcohol (e.g., ethanol-containing alcohol) and/or water (e.g., distilled water) and stirring it. Although not particularly limited, such washing can be repeated, for example, two or more times.

In an exemplary embodiment, the method may further include a drying step after the washing. Solvent residues, etc. may be removed through drying. The drying method is not particularly limited, and any known drying method may be used. For example, the drying may be performed using an oven or under room temperature conditions. Further, although not particularly limited, the drying may be performed in a vacuum.

In an exemplary embodiment, the method may further include a step of centrifuging, washing, and drying the suspension polymer after the completion of the suspension polymerization reaction. The description related to the washing and drying is the same as described above.

In addition, the particle composite of an embodiment may include particles having a coefficient of variation of the particle diameters of 20% or less, or 10% or less, or 9% or less, or 8% or less, or 7% or less, or 5% or less, or 3% or less, or 2% or less, or 0.1% or more, or 0.1% to 20%, or 0.1% to 10%, or 0.1% to 9%, or 0.1% to 8%, or 0.1% to 7%, or 0.1% to 5%, or 0.1% to 3%, or 0.1% to 2%.

The method of measuring the coefficient of variation of the particles is not limited, but can be obtained, for example, according to the following Mathematical Equation 2.

Coefficient of variation(%) = (Standard deviation of particle diameter / Average particle diameter) X 100 [Mathematical Equation 2]

The standard deviation of the particle diameter and average particle diameter in the Mathematical Equation 2 can be measured by analyzing SEM (scanning electron microscopy) images.

As the coefficient of variation of the particle diameter decreases to the above-mentioned range, the particles can be prepared with a uniform size, thereby maximizing particle performance.

On the other hand, if the coefficient of variation of the particle diameter increases excessively, such as more than 20%, the particle diameter deviation is large and uneven, which results in the generation of large particles, whereby there are problems that reproducibility is reduced during the preparation of cell therapeutic agents, and a separate classification

process is required, which reduces the preparation efficiency.

Meanwhile, the particles may be microfluidic chip particles. That is, the particles having a coefficient of variation of the particle diameter of 20% or less, or 10% or less, or 9% or less, or 8% or less, or 7% or less, or 5% or less, or 3% or less, or 2% or less, or 0.1% or more, or 0.1% to 20%, or 0.1% to 10%, or 0.1% to 9%, or 0.1% to 8%, or 0.1% to 7%, or 0.1% to 5%, or 0.1% to 3%, or 0.1% to 2% may be microfluidic chip particles. On the other hand, when the particles are suspension polymerized particles, the coefficient of variation of the particle diameter increases to more than 20%.

Specifically, the particle composite of an embodiment may be a particle composite comprising a spherical particle; and a protein immobilized on the particle surface, wherein the particle has a coefficient of variation of the particle diameter of 20% or less. In addition, the particle composite of an embodiment may be a particle composite comprising a spherical particle; and a protein immobilized on the particle surface, wherein the particle has a coefficient of variation of the particle diameter of 20% or less, and the particle may be a microfluidic chip particle.

More specifically, the microfluidic chip particle may be a particle obtained by a method for preparing particles comprising a) a step of injecting a dispersed phase composition containing a polymerizable monomer into a continuous phase composition through a microflow path to generate a liquid droplet composed of the dispersed phase composition within the continuous phase composition; and b) a step of photopolymerizing the liquid droplet.

In the step a) of generating a liquid droplet, a liquid droplet having a uniform size and shape may be prepared by a specific method.

In the step a) of generating a liquid droplet, as a dispersed phase composition forming a liquid droplet is injected into a continuous phase composition through a microflow path to generate a liquid droplet, it is possible to generate a liquid droplet having a uniform size.

Specifically, in the step a) of generating a liquid droplet, a microfluidic device including a microflow path may be used.

As an example, the microfluidic device may comprise: a first supply unit through which a dispersed phase composition is supplied; a first flow path through which the dispersed phase composition supplied from the first supply unit can flow; a second supply unit through which a continuous phase composition is supplied; a second flow path through which the continuous phase composition supplied from the second supply unit can flow; and a plurality of microflow paths connecting the side surfaces of the first and second flow paths. The side surface of a flow path refers to a direction other than the flow direction of the fluid flowing within the flow path.

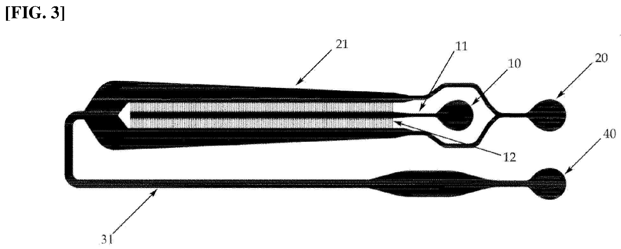

More specifically, referring to FIG. 3 the microfluidic device may include: a first supply unit 10 through which a dispersed phase composition is supplied; a first flow path 11 through which the dispersed phase composition supplied from the first supply unit can flow; a second supply unit 20 through which a continuous phase composition is supplied; a second flow path 21 through which the continuous phase composition supplied from the second supply unit can flow; and a plurality of microflow paths 12 connecting the side surfaces of the first and second flow paths. FIG. 3 is a diagram of a microfluidic device having a structure in which a first flow path 11 through which a dispersed phase composition flows is disposed between two second flow paths 21 through which a continuous phase composition flows, and both side surfaces of the first flow path 11 are connected to the side surfaces of the two second flow paths 21 through a plurality of microflow paths, which is an example in which a plurality of microflow paths are arranged as densely as possible. The structure of the microfluidic device is not limited to FIG. 3, and may be freely modified within a range that can achieve the purpose of the present disclosure with reference to FIG. 3.

The first flow path and the second flow path may be formed to be spaced apart from each other by the length of a desired microflow path. The height and length of the microflow path are not particularly limited, and may be appropriately adjusted according to the size of the desired liquid droplet.

In the step a) of generating a liquid droplet, a dispersed phase composition may be supplied to a first supply unit of a microfluidic device, and a continuous phase composition may be supplied to a second supply unit. The dispersed phase composition and the continuous phase composition may be injected into the first and second supply units, respectively, through a pump, but are not limited thereto.

The dispersed phase composition supplied to the first supply unit flows along the first flow path, and the continuous phase composition supplied to the second supply unit flows along the second flow path. In this case, the speed of the dispersed phase composition can be adjusted to 1 $\mu\ell$/min to 100 m$\ell$/min. The speed of the continuous phase composition can be adjusted to 10 $\mu\ell$/min to 500 m$\ell$/min.

The dispersed phase composition flowing through the first flow path flows into the second flow path through a plurality of microflow paths, and meets the continuous p hase composition flowing through the second flow path.

The dispersed phase composition flowing from the first flow path to the second flow path through the plurality of microflow paths generates a liquid droplet at the boundary between the microflow path and the second flow path, and the generated liquid droplet flows through the second flow path together with the continuous phase composition.

The microfluidic device may further include a discharge unit through which liquid droplet formed from the dispersed phase composition can be discharged. In this case, the microfluidic device may further include a third flow path 31 connecting the second flow path 21 and the discharge unit 40 as shown in FIG. 3.

The dispersed phase composition is a precursor composition for forming particles, and may be an oil phase that is insoluble in a continuous phase composition, which is a water phase.

Specifically, the dispersed phase composition may include a polymerizable monomer, a crosslinking agent, and a photoinitiator.

The polymerizable monomer may be a monomer having one or more unsaturated bonds. As an example, the polymerizable monomer may be a (meth)acrylate monomer or a (meth)acrylamide monomer having a (meth)acryloyl group, or a vinyl-based monomer having a vinyl group, or a mixture thereof.

Specifically, as a monomer containing an epoxy group, glycidyl methacrylate, glycidyl acrylate, and glycidyl acrylamide can be used alone or in a mixture with a monomer not containing an epoxy group.

The monomer not containing an epoxy group includes methyl methacrylate, methyl acrylate, ethyl acrylate, ethyl hexyl acrylate, butyl acrylate, hydroxyethyl methacrylate, trimethylolpropane triacrylate, N-isopropylacrylamide, N-butyl acrylate, and the like.

The crosslinking agent may form a crosslinked structure with a polymerizable monomer or a prepolymer formed therefrom, thereby allowing the particles to maintain a spherical shape. When the particles have a spherical shape, they may have excellent cell culture efficiency due to their large specific surface area. As an example, an ethylenically unsaturated crosslinking agent having two or more unsaturated bonds may be used as the crosslinking agent. More specifically, as the crosslinking agent, a multifunctional (meth)acrylate such as ethylene glycol di(meth)acrylate, trimethylolpropane tri(meth) acrylate, pentaerythritol tri(meth) acrylate or dipentaerythritol hexa(meth) acrylate may be used. Alternatively, one or more components among the crosslinking agents listed above may be used together. Considering the formation of a hydrophilic surface of the particle, etc., it may be preferable to use ethylene glycol di(meth)acrylate.

The content ratio of the crosslinking agent per 1 part by weight of the monomer may be 0.1 to 10 parts by weight, or 0.5 to 2 parts by weight. When the above content is satisfied, it is advantageous for stably forming an emulsion and securing particle strength and morphological characteristics through a desired level of crosslinking.

In an exemplary embodiment, the dispersed phase may be a solution in which glycidyl methacrylate(GMA) and ethylene glycol dimethacrylate(EGDMA) are mixed in a mass ratio of 5:1 to 1:5, or 4:1 to 1:4, or 3:1 to 1:3, or 2:1 to 1:2.

The type of the photoinitiator is not particularly limited, and various initiators known in the technical field to which the present disclosure belongs may be used as long as they do not become an obstacle to securing of the particle characteristics of the particles described above.

As the photoinitiator, for example, an initiator such as a ketone initiator, an organic peroxide initiator, or an azo initiator can be used. Specifically, compounds such as 2,2-dimethoxy-2-phenylacetophenone, 2-hydroxy-4'-(2-hydroxyethoxy)-2-methylpropiophenone, 1-hydroxycyclohexyl phenyl ketone, benzoyl peroxide, di-t-amyl peroxide, t-butyl peroxybenzoate, 2,5-dimethyl-2,5 di-(t-butylperoxy)hexane, 2,5-dimethyl-2,5-di-(t-butylperoxy)hexyne-3 or di-cumyl peroxide, and mixtures thereof can be used, but are not limited thereto.

The photoinitiator may be included in an amount of 0.1 parts by weight or more, 1 part by weight or more, 3 parts by weight or more, 5 parts by weight or more, 10 parts by weight or more, or 15 parts by weight or more, and 30 parts by weight or less, 25 parts by weight or less, 20 parts by weight or less, or 18 parts by weight or less, based on 100 parts by weight of the polymerizable monomer.

Within this range, microparticles exhibiting an appropriate polymerization rate and desired properties can be prepared.

In addition to the above components, the dispersed phase composition may include various additives known in the technical field to which the present disclosure belongs, within the scope that does not interfere with the purpose of the present disclosure.

Meanwhile, the continuous phase composition may be a water phase. Specifically, the continuous phase composition may be an aqueous solution containing a surfactant and water.

The water is not particularly limited, but may be distilled water or deionized water.

The surfactant may be an ionic surfactant or a nonionic surfactant. For example, the ionic surfactant may be sodium dodecyl sulfate(SDS), and the nonionic surfactant may be Tween 20, Tween 40, Tween 60, Tween 80, Triton X-100, polyvinyl alcohol(PVA), polyethylene glycol(PEG), and the like.

The continuous phase composition may contain the surfactant in an amount of 0.01 wt.% or more, 0.05 wt.% or more, 0.1 wt.% or more, 0.2 wt.% or more, or 0.3 wt.% or more, and 5 wt.% or less, 3 wt.% or less, or 1 wt.% or less, based on the total continuous phase composition. It is advantageous to prepare microparticles having desired characteristics within this range.

Meanwhile, the liquid droplet prepared in the step a) of generating a liquid droplet may be introduced to the step b) of photopolymerizing the liquid droplet. In this case, the liquid droplet may be mixed with an additional continuous phase composition before being introduced to the photopolymerization process.

As an example, a suspension including the liquid droplet discharged through the discharge port of the microfluidic device may be mixed with an additional continuous phase composition and then introduced to the photopolymerization

process. Through this process, the shape of the microparticles may be uniformly controlled.

The additional continuous phase composition may be the same composition as or different from the continuous phase composition used in the step a) of generating a liquid droplet. As an example, in the step a) of generating a liquid droplet, an aqueous solution of sodium dodecyl sulfate may be used as the continuous phase composition, and the suspension containing the liquid droplet may be mixed with an aqueous solution of polyvinyl alcohol. However, without being limited thereto, the liquid droplet generated in step a) of generating a liquid droplet may be directly transferred to a photopolymerization process.

In the photopolymerization step b), the liquid droplet generated in step a) are photopolymerized to prepare a particle. In the photopolymerization step b), photopolymerization of the liquid droplet can be induced by irradiating the liquid droplet with UV light using a UV spot curing device. The specific conditions of the photopolymerization are not particularly limited, and various exemplary embodiments and conditions widely used in the conventional particle preparation field can be applied without limitation.

After the photopolymerization step b), a washing step may be further included. Impurities unrelated to the particles can be removed by washing. The washing method is not particularly limited, and a known washing method can be used. For example, the washing can be performed by adding the particles to alcohol such as ethanol and stirring them. Although not particularly limited, this washing can be repeated, for example, three or more times.

After the washing, a drying step can be further included. Residual solvents, and the like can be removed through drying. The drying method is not particularly limited, and a known drying method can be used. For example, the drying can be performed using an oven or under room temperature conditions. Further, although not particularly limited, the drying can be performed in a vacuum.

According to the preparation method of particles, it is possible to prepare particles having a uniform size and shape. In the case of conventional preparation methods of particles, they goes through the process of sorting particles having desired shapes and properties from particles having undesired shapes or properties after particle formation, which results in a low preparation yield. However, the preparation method of particles can obtain particles having a desired shape and properties without going through this sorting process, thereby exhibiting a high preparation yield.

Meanwhile, the particle composite of an embodiment can include a protein immobilized on the surface of the particle.

The protein can be a protein for immune cell differentiation. The protein for immune cell differentiation can play a role in providing a stimulus required for differentiating stem cells such as allogeneic induced pluripotent stem cells (iPSCs) into immune cells.

Specific examples of the protein for immune cell differentiation are not particularly limited, and examples thereof include GM-CSF, IL4, IL-1b, TNFa, PGE2, DLL1, DLL4, TGFb, or a mixture of two or more thereof. In addition, well-known proteins can be applied without limitation.

Since the protein has the function of differentiating immune cells, at the time of culturing the particle composite of an embodiment together with stem cells, the stem cells can be differentiated into immune cells to prepare a cell therapeutic agent for anticancer treatment, and the like. By the introduction of such particles, the technical limitations of surface protein adsorption used in the existing immune cell differentiation platform can be effectively overcome, thereby making it possible to economically produce large amounts of immune cells and have high marketability as an off-the-shelf drug.

The protein content per 1g of the particle may be 0.05 mg to 10 mg. If the protein content per 1g of the particle is excessively reduced outside the above numerical range, it is difficult to sufficiently achieve stem cell differentiation by the protein. On the other hand, if the protein content per 1g of the particles is excessively increased outside the above numerical range, the protein may become higher than the density of the ligand for immobilizing on the cell surface, which may cause a technical problem that proteins that cannot be immobilized are generated. .

The protein may be immobilized on the particle surface by a covalent bond or a physical bond. The particle composite of an embodiment may secure structural stability by immobilizing the protein to the particle surface by a chemical bond (e.g., covalent bond) or a physical bond (e.g., adsorption). In particular, if the protein is immobilized to the particle surface by a chemical bond (e.g., covalent bond), mass production and automation can be stably performed through a 3D process compared to conventional technologies, thereby increasing the efficiency of the process.

The covalent bond is a bond that is generated when atoms share electrons during the chemical bond, and the protein can be immobilized to the surface of the particle by a covalent bond in a state where the particle is dispersed in water.

Specifically, the covalent bond may include a bonding functional group represented by the following Chemical Formula 1:

[Chemical Formula 1]

wherein in Chemical Formula 1, $R_1$ is hydrogen or an alkyl.

That is, the covalent bond may have a structure in which a particle and a protein are bonded via a bonding functional group represented by the Chemical Formula 1. More specifically, a particle may be bonded to one end of the bonding functional group represented by the Chemical Formula 1, and a protein may be bonded to the other end of the bonding functional group represented by the Chemical Formula 1.

As an example, the covalent bond may include a bond represented by the following Chemical Formula 2.

[Chemical formula 2]

wherein in Chemical Formula 2, $R_1$ is hydrogen or an alkyl, X is a particle, and Y is a protein.

The covalent bond represented by the Chemical Formula 2 can be formed by a reaction between an epoxy group on the particle surface and an amine group contained in the protein. The reaction conditions between the epoxy group on the particle surface and the amine group contained in the protein are not particularly limited, and various well-known covalent bond conditions can be applied without limitation. However, as an example, ammonium sulfate can be added to a solution in which a particle and a protein are mixed in PBS (phosphate buffered saline), and the reaction can be performed for 16 hours or more while shaking at a speed of 250 rpm or more at room temperature.

Further, the covalent bond can include a binding functional group represented by the following Chemical Formula 3.

[Chemical Formula 3]

*
S
*
OH

That is, the covalent bond may have a structure in which a particle and a protein are bonded via a bonding functional group represented by the Chemical Formula 3. More specifically, a particle may be bonded to one end of the bonding functional group represented by the Chemical Formula 3, and a protein may be bonded to the other end of the bonding functional group represented by the Chemical Formula 3.

As an example, the covalent bond may include a bond represented by the following Chemical Formula 4.

[Chemical Formula 4]

Y
S
X
OH

wherein in Chemical Formula 4, X is a particle, and Y is a protein.

The covalent bond represented by the Chemical Formula 4 can be formed by a reaction between an epoxy group on the particle surface and a thiol group contained in the protein. The reaction conditions between the epoxy group on the particle surface and the thiol group contained in the protein are not particularly limited, and various well-known covalent bond conditions can be applied without limitation. However, as an example, ammonium sulfate can be added to a solution in which a particle and a protein are mixed in PBS (phosphate buffered saline), and the reaction can be performed for 16 hours or more while shaking at a speed of 250 rpm or more at room temperature.

Further, the covalent bond can include a binding functional group represented by the following Chemical Formula 5.

[Chemical Formula 5]

O
*
N
*
R2

wherein in Chemical Formula 5, $R_2$ is hydrogen or an alkyl.

That is, the covalent bond may have a structure in which a particle and a protein are bonded via a bonding functional group represented by the Chemical Formula 5. More specifically, a particle may be bonded to one end of the bonding functional group represented by the Chemical Formula 5, and a protein may be bonded to the other end of the bonding functional group represented by the Chemical Formula 5.

As an example, the covalent bond may include a bond represented by the following Chemical Formula 6.

[Chemical Formula 6]

Y
O
N
R2
X

wherein in Chemical Formula 6, $R_2$ is hydrogen or an alkyl, X is a particle, and Y is a protein.

The covalent bond represented by the Chemical Formula 6 can be formed by a reaction between a carboxyl group on the particle surface and an amine group contained in the protein. The reaction conditions between the carboxyl

group on the particle surface and the amine group contained in the protein are not particularly limited, and various well-known covalent bond conditions can be applied without limitation. However, as an example, ammonium sulfate can be added to a solution in which a particle and a protein are mixed in PBS (phosphate buffered saline), and the reaction can be performed for 16 hours or more while shaking at a speed of 250 rpm or more at room temperature.

Meanwhile, since the particle composite of an embodiment has the function of differentiating immune cells, at the time of culturing the particle composite of an embodiment together with stem cells, the stem cells can be differentiated into immune cells to prepare a cell therapeutic agent for anticancer treatment, and the like. By the introduction of such particles, the technical limitations of surface protein adsorption used in the existing immune cell differentiation platform can be effectively overcome, thereby making it possible to economically prepare large amounts of immune cells and have high marketability as an off-the-shelf drug.

The particle composite of an embodiment has a liberation protein ratio of 0.1% or less, or 0.0001% to 0.1%, according to the following Mathematical Equation 1.

[Mathematical Equation 1]

$$\text{Liberation protein ratio (\%)} = (W1 / W2) * 100$$

in Mathematical Equation 1, W1 is the mass of a protein liberated from the particle composite after shaking culture and centrifugation of the solution containing the particle composite, and W2 is the mass of a protein immobilized to the particle composite before shaking culture and centrifugation of the solution containing the particle composite.

This is considered to be because the particle composite of an embodiment immobilizes the protein to the particle surface through covalent bond to ensure structural stability. Thereby, compared to the conventional technology, mass production and automation can be stably performed through the 3D process, thereby increasing the efficiency of the process.

On the other hand, if the liberation protein ratio according to Mathematical Equation 1 increases excessively by more than 0.1%, the protein may not be stably immobilized from the particle composite, which may cause a problem that the efficiency of the process is significantly reduced when mass production and automation are performed through the 3D process.

The solution containing the particle composite is a mixture of the particle composite and the solvent, and examples of the solvent are not particularly limited, and various well-known solvents can be applied without limitation. However, as an example, PBS (Phosphate buffered saline) can be used. The particle composite can be added so that the protein concentration in the solution containing the particle composite satisfies 1 ug/mL.

Examples of the shaking culture conditions are not particularly limited, and various well-known shaking culture conditions can be applied without limitation. However, as an example, the reaction can be performed for 7 hours or more while shaking at a speed of 250 rpm or more in a shaking incubator at room temperature.

Examples of the centrifugation conditions are not particularly limited, and various well-known centrifugation conditions can be applied without limitation. However, as an example, centrifugation can be performed at 10,000 xg for 10 minutes.

Examples of the method for measuring the mass of protein liberated from the particle composite are not particularly limited, and various well-known quantitative analysis methods can be applied without limitation. However, as an example, ELISA (Enzyme-Linked Immunosorbent Assay) can be mentioned.

Meanwhile, the particle composite of an embodiment may comprise a spherical particle; and a protein immobilized on the surface of the particle. The contents related to the spherical particle and the content of the protein immobilized on the particle surface are the same as described above. If the particle composite includes other components (e.g., magnetic particles) in addition to the spherical particle and the protein immobilized on the particle surface, it is difficult to remove them from the particle composite, and thus to maintain the quality, and there is a limitation in that a separate removal process is required for reuse, which reduces process efficiency.

However, if necessary, the particle composite of an embodiment may further include magnetic particles. Specifically, if the magnetic particles are further included in the particles of the particle composite, the particle composite and cells can be separated and purified more easily using magnetism when separating and recovering them after cell culture.

The magnetic particles refer to particles that exhibit magnetic properties. All materials interact with a magnetic field to generate an attractive force or a repulsive force. That is, when a magnetic field is applied to a material, it becomes magnetized, and is classified into a ferromagnetic material, a paramagnetic material, a semimagnetic material, a ferrimagnetic material, and the like, depending on the pattern in which an object is magnetized.

The magnetic particles may be prepared by solution synthesis, co-precipitation, sol-gel processes, high-energy grinding, hydrothermal synthesis, microemulsion synthesis, synthesis by thermal decomposition, or sonochemical synthesis, but are not limited thereto.

The type of the magnetic particles is not particularly limited, and metal nanoparticles may be used. For example, the magnetic particles may include one or more metals selected from the group consisting of gold(Au), silver(Ag), cobalt(Co), copper(Cu), iron(Fe), chromium(Cr), nickel(Ni), palladium(Pd), platinum(Pt), and tin(Sn) , or oxides thereof. Specifically, the magnetic particles may include iron(Fe) particles.

**2. Cell culture composition**

According to another embodiment of the present disclosure, there can be provided a cell culture composition comprising a cell and the particle composite of an embodiment. The contents related to the particle composite includes all the contents described above in the above embodiment.

The cell may include stem cells.

The cell culture composition may further include a medium solution. The medium solution may include various additives to sufficiently satisfy environmental conditions such as pH, temperature, and osmotic pressure and nutrients similar to the conditions of a living body based on body fluids such as plasma or lymph, and various materials widely known in the art related to cell culture may be used without limitation.

**3. Method for preparing a cell therapeutic agent**

According to another embodiment of the present disclosure, there can be provided a preparation method for a cell therapeutic agent, the method comprising the steps of: culturing the cell culture composition of another embodiment described above; and removing the particle composite from the culture result. The contents related to the cell culture composition include all the contents described above in the other embodiments.

In the step of culturing the cell culture composition of the other embodiments, the cells included in the cell culture composition can be differentiated into immune cells. The specific culture conditions are not particularly limited, and culture conditions that can differentiate stem cells into immune cells can be applied without limitation.

Meanwhile, in the step of removing the particle composite from the culture result, the safety of the cell therapeutic agent can be secured by separating and removing the particle composite from the result of the cell culture step. The method of removing the particle composite from the culture result is not particularly limited, and various methods and conditions well-known in the art can be applied without limitation. However, as an example, the particle composite can be separated and removed through a cell strainer.

**[Advantageous Effects]**

According to the present disclosure, a particle composite that not only can immobilize proteins required for cell differentiation to ensure the stability of the differentiation platform but also can be completely separated after cell culture to ensure the stability of a cell therapeutic agent, and a cell culture composition and a preparation method for a cell therapeutic agent using the same can be provided.

**[BRIEF DESCRIPTION OF THE DRAWING]**

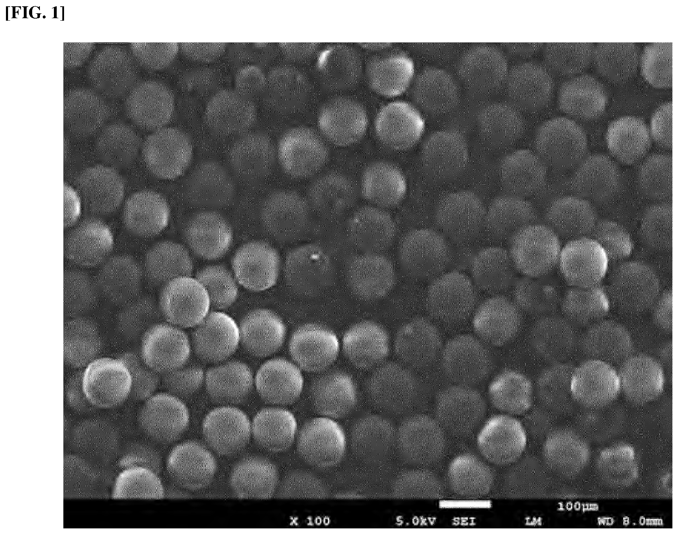

FIG. 1 shows an SEM image of the particles obtained in (1) of Example 1.

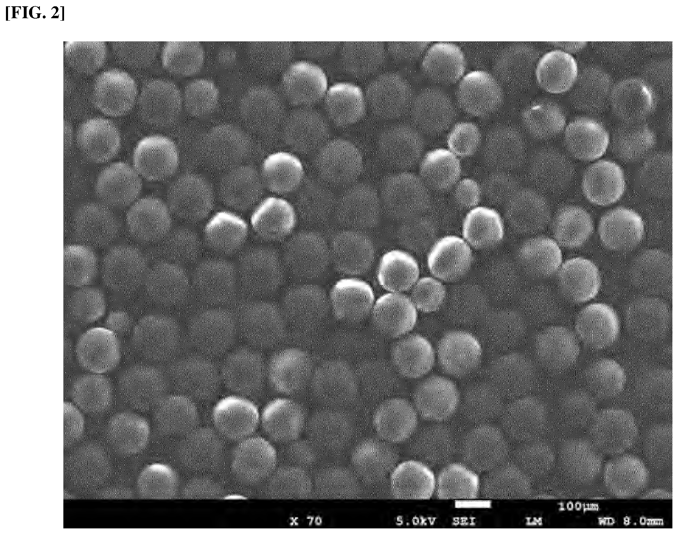

FIG. 2 shows an SEM image of the particles obtained in (1) of Example 6.
FIG. 3 is a plan view of a microfluidic device.

**[DETAILED DESCRIPTION OF THE EMBODIMENTS]**

Hereinafter, the present disclosure will be described in more detail with reference to examples. However, the following examples are provided for illustrative purposes only, and are not intended to limit the scope of the present disclosure,

**<Example: Preparation of particle composite>**

<u>Example 1</u>

(1) Preparation of particle

GMA (glycidyl methacrylate) as a monomer and EGDMA (ethylene glycol dimethacrylate) as a crosslinking agent were stirred, and V-65 as a thermal initiator was further added, and stirred at room temperature for about 5 minutes to prepare a dispersed phase. In this case, the weight ratio of monomer: crosslinking agent: initiator was 1:3:0.05.

PVA (polyvinyl alcohol; weight average molecular weight(Mw): 85,000 to 124,000, hydrolysis rate satisfying 87 to 89%) was dissolved in distilled water at a concentration of 2 wt.% to prepare a continuous phase.

720 g of the resulting continuous phase was added to a reactor with a predetermined capacity, and 72 g of the dispersed phase was added thereto, and stirred at about 800 rpm at room temperature until a uniform dispersion was obtained. The reactor temperature was then increased, and polymerization was performed at a temperature of about 80°C, about 800 rpm and under nitrogen purging conditions for about 6 hours.

The polymerized particles were recovered and washed twice with distilled water and five times with ethanol. The result was then dried in an oven at about 80°C to prepare a particle. The D50 diameter of the prepared particle was measured to be 130 μm (wherein the D50 means the size of the 50th particle when a SEM (scanning electron microscope) image is analyzed and the total number of particles is calculated to be 100.)

(2) Preparation of particle composite

The particles obtained in (1) were washed three times with PBS (phosphate buffered saline) by centrifuging at 10,000 xg for 10 minutes. Then, proteins for immune cell differentiation (GM-CSF, IL4, IL-1b, TNFa, PGE2, DLL1, DLL4, TGFb, etc., each of which were applied independently) were dissolved in PBS and mixed at a ratio of 0.05 mg to 10 mg per 1 g of the particles.

The PBS solution containing the particles obtained in (1) and the protein for immune cell differentiation, and 3M ammonium sulfate were added in the same volume so that the final solution was made to be 1.5 M ammonium sulfate. Then, the resulting solution was reacted for 16 hours or more while shaking at a speed of 250 rpm or more at room temperature in a shaking incubator, so that the epoxy group exposed on the surface of the particles obtained in (1) was covalently bonded to the amine or thiol group of the protein.

Then, centrifugation was performed at 10,000 xg for 10 minutes, the particles with covalently bound proteins were separated from the mixed solution, the supernatant was removed, and then pH 12 Tris buffer was added and voltexed. The buffer solution was removed by centrifugation, pH 4 acetate buffer was added and vortexed. The particles were then washed three more times with PBS containing 0.5 wt.% of Tween 20, and the resulting particles with covalently bound proteins were used as the particle composite of Example 1. The particle composite was placed in PBS and stored at 4°C.

Example 2

(1) Preparation of particle

A particle was prepared in the same manner as in (1) of Example 1, except that the D50 diameter of the particle was changed to 100 μm.

(2) Preparation of particle composite

A particle composite was prepared in the same manner as in (2) of Example 1, except that the particle obtained in (1) of Example 2 was used instead of the particle obtained in (1) of Example 1.

Example 3

(1) Preparation of particle

A particle was prepared in the same manner as in (1) of Example 1, except that the D50 diameter of the particle was changed to 50 μm.

(2) Preparation of particle composite

A particle composite was prepared in the same manner as in (2) of Example 1, except that the particle obtained in (1) of Example 3 was used instead of the particle obtained in (1) of Example 1.

Example 4

(1) Preparation of particle

A particle was prepared in the same manner as in (1) of Example 1, except that the D50 diameter of the particle was changed to 38 μm.

(2) Preparation of particle composite

A particle composite was prepared in the same manner as in (2) of Example 1, except that the particle obtained in (1) of Example 4 was used instead of the particle obtained in (1) of Example 1.

Example 5

(1) Preparation of particle

Glycidyl methacrylate(GMA) as a monomer and ethylene glycol dimethacrylate(EGDMA) as a crosslinking agent were stirred in a weight ratio of GMA: EGDMA = 1:1, and then Irgacure 651 as a photoinitiator was further added at 1 wt.% based on the weight of the stirred solution, and stirred at room temperature for about 5 minutes to prepare a dispersed phase composition.

SDS(sodium dodecyl sulfate) was dissolved in deionized water at a concentration of 0.5 wt.% to prepare a continuous phase composition.

The previously prepared dispersed phase composition (injection rate of 100 μl/min) and continuous phase composition (injection rate of 300 μℓ/min) were respectively injected through a pump into the first supply unit 10 and the second supply unit 20 of the microfluidic device (40 μm step emulsification chip) as shown in FIG. 3 below. The dispersed phase composition injected into the first supply unit 10 flowed along the first flow path 11 and was supplied to the second flow path 21 through the microflow path 12, thereby forming a liquid droplet in the continuous phase composition. The suspension containing the liquid droplet was discharged through the discharge unit 40.

The suspension containing the liquid droplet was then collected in an aqueous solution of PVA (polyvinyl alcohol; molecular weight: 85,000~125,000) so that the liquid droplet was dispersed in a final 3 wt.% PVA. After shaking to disperse the liquid droplet evenly, photopolymerization was performed using a UV spot curing device.

The polymerized particle was recovered, and washed twice with distilled water and five times with ethanol. The result was then dried in an oven at about 80°C to prepare a particle.

(2) Preparation of particle composite

A particle composite was prepared in the same manner as in (2) of Example 1, except that the particle obtained in (1) of Example 5 was used instead of the particle obtained in (1) of Example 1.

Example 6

(1) Preparation of particle

A particle was prepared in the same manner as in (1) of Example 5, except that the following four conditions were changed.

1) GMA: EGDMA = weight ratio of 1:2
2) Dispersed phase injection rate: 10 μℓ/min
3) Continuous phase injection rate: 15 μℓ/min
4) Microfluidic device: 100 μm flow focusing chip

(2) Preparation of particle composite

A particle composite was prepared in the same manner as in (2) of Example 1, except that the particle obtained in (1) of Example 6 was used instead of the particle obtained in (1) of Example 1.

Example 7

(1) Preparation of particle

A particle was prepared in the same manner as in (1) of Example 5, except that the following four conditions were changed.

1) GMA: EGDMA = weight ratio of 1:2
2) Dispersed phase injection rate: 15 μL/min
3) Continuous phase injection rate: 225 μL/min
4) Microfluidic device: 190 μm flow focusing chip

(2) Preparation of particle composite

A particle composite was prepared in the same manner as in (2) of Example 1, except that the particle obtained in (1) of Example 7 was used instead of the particle obtained in (1) of Example 1.

**<Comparative Example>**

Comparative Example 1

Using a flat-bottom tissue culture plate (the polystyrene culture plate, which is a form in which proteins are immobilized directly to the bottom without any particulate support), proteins for immune cell differentiation (GM-CSF, IL4, IL-1b, TNFa, PGE2, DLL1, DLL4, TGFb, etc.) were diluted to the target concentration in PBS at 4°C, and then added to a 96-well plate at 50 uL/well. They were stored flat at 4°C for 16 hours or more to induce adsorption, and then washed twice with PBS to remove residual proteins, thereby preparing a protein composite.

**<Reference Example: Preparation of particle composite>**

Reference Example 1

Commercially available particles (D50 diameter 180 μm) were prepared in PBS at a concentration of 0.1 mg/mL, and proteins for immune cell differentiation (GM-CSF, IL4, IL-1b, TNFa, PGE2, DLL1, DLL4, TGFb, etc.) were added at 0.05 mg to 5 mg per 1 g of particles. The reaction was then performed for 16 hours or more while shaking at a speed of 250 rpm or more at room temperature, and then the reaction product was washed three times with PBS to remove residual proteins, thereby preparing a particle composite.

**Reference Example 2**

5 mg of commercially available magnetic beads (D50 diameter 5 μm) were dispersed in 1 mL of PBS, and the supernatant was removed using a magnet. 1 ug to 100 ug of proteins for immune cell differentiation (GM-CSF, IL4, IL-1b, TNFa, PGE2, DLL1, DLL4, TGFb, etc.) were added so as to make 1 mg/mL. The total solution and 3 M ammonium sulfate solution were added in the same volume, and tilt rotation was slowly performed at 37°C for 16 hours or more. The supernatant was removed using a magnet, and then washed four times with PBS to prepare a particle composite.

**<Experimental Example 1>**

The physical properties of the composites obtained in Examples, Comparative Examples, and Reference Examples were measured using the following method, and the results are shown in Table 1.

**1. Protein Stability**

The composites obtained in Examples, Comparative Examples, and Reference Examples were dissolved in PBS at 1 ug/mL based on protein in a 1 mL EP tube, and reacted for 7 hours or more while shaking at a speed of 250 rpm or more at room temperature in a shaking incubator. The supernatant was then collected by centrifugation at 10,000 xg for 10 minutes. Then, ELISA (Enzyme-Linked Immunosorbent Assay) was performed with the supernatant, and the mass of the protein liberated from the particle composite was measured, and the liberation protein ratio was calculated according to the following Mathematical Equation 1, and the protein stability was evaluated under the following criteria.

[Mathematical Equation 1]

$$\text{Liberation protein ratio (\%)} = (W1 / W2) * 100$$

in Mathematical Equation 1,

W1 is the mass of a protein liberated from the particle composite after shaking culture and centrifugation of the solution containing the particle composite, and
W2 is the mass of a protein immobilized to the particle composite before shaking culture and centrifugation of the solution containing the particle composite.

[Evaluation criteria]

Upper: Liberation protein ratio according to Mathematical Equation 1 is 0.1% or less
Middle: Liberation protein ratio according to Mathematical Equation 1 is more than 0.1% and 10% or less
Lower: Liberation protein ratio according to Mathematical Equation 1 is more than 10%

**2. Particle separation performance**

A 10 mL vial was filled with a culture medium containing cells, and the composites obtained in Examples, Comparative Examples, and Reference Examples were injected and mixed for 30 minutes using a rocking shaker. After separating the particle composite from the cell culture medium using a cell strainer, whether the particle composite remained in the cell culture medium was analyzed using a cell culture microscope and a particle size analyzer (Beckman Coulter counter).
The particle separation performance of the particle composite was evaluated under the following criteria.
Upper: The residual amount of particle composite is less than 0.1% of the feed amount.
Middle: The residual amount of particle composite is 0.1% or more and less than 5% of the feed amount.
Lower: The residual amount of particle composite is 5% or more of the feed amount.

[Table 1]

| Measurement results of Experimental Example 1 | | | | | | | | | | |
|---|---|---|---|---|---|---|---|---|---|---|
| | Example 1 | Example 2 | Example 3 | Example 4 | Example 5 | Example 6 | Example 7 | Comparative Example 1 | Reference Example 1 | Reference Example 2 |
| Particle shape | Spherica 1 | Spherica 1 | Spherica 1 | Spherica 1 | Spherica 1 | Spherica 1 | Spherica 1 | - (not particle) | Spherica 1 | Spherica 1 |
| Particle diameter (D50) | 130 μm | 100 μm | 50 μm | 38 μm | 50 μm | 100 μm | 130 μm | - | 180 μm | 5 μm |
| Protein stability | Upper | Upper | Upper | Upper | Upper | Upper | Upper | Lower | Middle | Upper |
| Particle separation performance | Upper | Upper | Upper | Middle | Upper | Upper | Upper | - (not particle) | Upper | Lower |

As shown in Table 1, it was confirmed that in the particle composites of Examples, the proteins were stably immobilized under cell culture conditions compared to Comparative Examples, and were completely separated after cell culture.

**<Experimental Example 2>**

The physical properties of the composites obtained in Examples 1 and 5~7 were measured using the following method, and the results are shown in Table 2.

**1. Particle diameter and coefficient of variation**

(1) Particle diameter

The particles obtained in Examples 1, 5 to 7 were observed by analyzing SEM (scanning electron microscope) images, and the particle diameters were measured.
The D50 diameter was measured as the size of the 50th particle when the total number of particles was converted to 100.

(2) Coefficient of variation (CV)

For the 100 particle samples obtained in Examples 1, 5 to 7, the SEM (scanning electron microscopy) images were analyzed and the coefficient of variation was obtained according to the following Mathematical Equation 2.

Coefficient of variation(%) = (Standard deviation of particle diameter / Average particle diameter) X 100. [Mathematical Equation 2]

[Table 2]

| Measurement results of Experimental Example 2 | | | | |
|---|---|---|---|---|
| | Example 1 | Example 5 | Example 6 | Example 7 |
| GMA : EGDMA | 1:3 | 1:1 | 1:2 | 1:2 |
| Polymerization condition | Suspension polymerization | Photopolymerization<br>1) Dispersed phase injection rate: 100 μℓ/min<br>2) Continuous phase injection rate: 300 μℓ/min<br>3) Microfluidic device: 40 μm step emulsification chip | Photopolymerization<br>1) Dispersed phase injection rate: 10 μℓ /min<br>2) Continuous phase injection rate: 15 μℓ /min<br>3) Microfluidic device: 100 μm flow focusing chip | Photopolymerization<br>1) Dispersed phase injection rate: 15 μℓ /min<br>2) Continuous phase injection rate: 225 μℓ/min<br>3) Microfluidic device: 190 μm flow focusing chip |
| Particle diameter | 135.9 μm ± 28 μm | 53.1 μm ± 4.1 μm | 99.0 μm ± 2.2 μm | 131.0 μm ± 2.8 μm |
| Particle diameter (D50) | 130 μm | 50 μm | 100 μm | 130 μm |
| Coefficient of variation | 21% | 7.7% | 2.2% | 2.1% |

**Claims**

**1.** A particle composite comprising:

a spherical particle; and
a protein immobilized on the particle surface.

**2.** The particle composite of claim 1, wherein the particle has a D50 diameter of more than 20 $\mu$m.

**3.** The particle composite of claim 1, wherein the particle has a D50 diameter of less than 180 $\mu$m.

**4.** The particle composite of claim 1, wherein the particle has a D50 diameter of 30 $\mu$m to 175 $\mu$m.

**5.** The particle composite of claim 1, wherein the particle has a D50 diameter of 50 $\mu$m to 160 $\mu$m.

**6.** The particle composite of claim 1, wherein the protein is immobilized on the particle surface by a covalent bond or a physical bond.

**7.** The particle composite of claim 6,

wherein the covalent bond includes a bonding functional group represented by the following Chemical Formula 1:

[Chemical Formula 1]

wherein in Chemical Formula 1, $R_1$ is hydrogen or an alkyl.

**8.** The particle composite of claim 7,

wherein the covalent bond includes a bond represented by the following Chemical Formula 2:

[Chemical Formula 2]

Y
X
N
R1
OH

wherein in Chemical Formula 2, $R_1$ is hydrogen or an alkyl, X is a particle, and Y is a protein.

**9.** The particle composite of claim 6, wherein the covalent bond includes a bonding functional group represented by the following Chemical Formula 3:

[Chemical Formula 3]

**10.** The particle composite of claim 9,

wherein the covalent bond includes a bond represented by the following Chemical Formula 4:

[Chemical Formula 4]

Y
X
S
OH

wherein in Chemical Formula 4,
X is a particle, and Y is a protein.

**11.** The particle composite of claim 6,

wherein the covalent bond includes a bonding functional group represented by the following Chemical Formula 5:

[Chemical Formula 5]

O
N
*
*
$R_2$

wherein in Chemical Formula 5, $R_2$ is hydrogen or an alkyl.

**12.** The particle composite of claim 11,

wherein the covalent bond includes a bond represented by the following Chemical Formula 6:

[Chemical Formula 6]

wherein in Chemical Formula 6, $R_2$ is hydrogen or an alkyl,
X is a particle, and Y is a protein.

**13.** The particle composite of claim 1,
wherein the protein content per 1g of the particle is 0.05 mg to 10 mg.

**14.** The particle composite of claim 1,
wherein the protein is a protein for immune cell differentiation.

**15.** The particle composite of claim 1,
wherein the particle is a suspension-polymerized particle.

**16.** The particle composite of claim 1,
wherein the particle has a coefficient of variation of the particle diameter of 20% or less.

**17.** The particle composite of claim 16,
wherein the particle is a microfluidic chip particle.

**18.** The particle composite of claim 1,
wherein the particle composite has a liberation protein ratio of 0.1% or less according to the following Mathematical Equation 1.

[Mathematical Equation 1]

$$\text{Liberation protein ratio (\%)} = (W1 / W2) * 100$$

in Mathematical Equation 1,

W1 is the mass of a protein liberated from the particle composite after shaking culture and centrifugation of the solution containing the particle composite, and
W2 is the mass of a protein immobilized to the particle composite before shaking culture and centrifugation of the solution containing the particle composite.

**19.** A cell culture composition comprising a cell and the particle composite of claim 1.

**20.** A preparation method for a cell therapeutic agent, the method comprising the steps of:

culturing the cell culture composition of claim 19; and
removing the particle composite from the culture result.

[FIG. 1]

100μm
X 100
5.0kV
SEI
LM
WD 8.0mm

[FIG. 2]

100µm
X 70
5.0kV SEI
LM
WD 8.0mm

[FIG. 3]

21
11
10
20
40
12
31

## INTERNATIONAL SEARCH REPORT

International application No. **PCT/KR2024/017375**

**A. CLASSIFICATION OF SUBJECT MATTER**

**C07K 17/06**(2006.01)i; **C12N 5/074**(2010.01)i; **A61K 35/17**(2015.01)i

According to International Patent Classification (IPC) or to both national classification and IPC

**B. FIELDS SEARCHED**

Minimum documentation searched (classification system followed by classification symbols)

C07K 17/06(2006.01); A61K 47/60(2017.01); A61K 47/69(2017.01); B01J 19/10(2006.01); C07K 1/34(2006.01); C12N 5/0783(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 입자 표면 (particle surface), 입자 복합체 (particle complex), 단백질 (protein), 세포 치료제 (cell therapy agent)

**C. DOCUMENTS CONSIDERED TO BE RELEVANT**

| Category* | Citation of document, with indication, where appropriate, of the relevant passages | Relevant to claim No. |
|---|---|---|
| | ZHU, X. H. et al. Proteins combination on PHBV microsphere scaffold to regulate Hep3B cells activity and functionality: A model of liver tissue engineering system. Journal of Biomedical Materials Research Part A. 2007, vol. 83A, pp. 606-616. | |
| X | See abstract; pages 607 and 608; and figure 1(a). | 1-6,13-16,18,19 |
| Y | | 7-10,20 |
| A | | 11,12,17 |
| | KR 10-2017-0047622 A (AMOLIFESCIENCE CO., LTD.) 08 May 2017 (2017-05-08) | |
| X | See claims 1 and 5; paragraphs [0004], [0018], [0144] and [0154]; and figure 1. | 1,3,6,11-13,15-18 |
| Y | | 7-10 |
| | KR 10-2017-0134318 A (NEXIMMUNE, INC.) 06 December 2017 (2017-12-06) | |
| Y | See paragraph [0065]. | 7-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2025** | **20 February 2025** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
**PCT/KR2024/017375**

**C. DOCUMENTS CONSIDERED TO BE RELEVANT**

| Category* | Citation of document, with indication, where appropriate, of the relevant passages | Relevant to claim No. |
|---|---|---|
| Y | KR 10-2020-0132147 A (SUNGKWANG MEDICAL FOUNDATION et al.) 25 November 2020 (2020-11-25)<br>See claims 1, 9, 15 and 16; and paragraphs [0004] and [0005]. | 20 |
| A | US 2013-0337528 A1 (THOMPSON, T. et al.) 19 December 2013 (2013-12-19)<br>See paragraph [0014]. | 1-20 |

Form PCT/ISA/210 (second sheet) (July 2022)

**INTERNATIONAL SEARCH REPORT**
**Information on patent family members**

International application No.
**PCT/KR2024/017375**

| Patent document cited in search report | Publication date (day/month/year) | Patent family member(s) | Publication date (day/month/year) |
|---|---|---|---|
| KR 10-2017-0047622 A | 08 May 2017 | CN 108139398 A | 08 June 2018 |
| | | KR 10-1966360 B1 | 16 April 2019 |
| | | US 11169147 B2 | 09 November 2021 |
| | | US 2018-0313824 A1 | 01 November 2018 |
| | | WO 2017-069583 A1 | 27 April 2017 |
| KR 10-2017-0134318 A | 06 December 2017 | AU 2015-371265 A1 | 13 July 2017 |
| | | AU 2015-371265 B2 | 03 June 2021 |
| | | AU 2021-225200 A1 | 30 September 2021 |
| | | BR 112017013838 A2 | 19 June 2018 |
| | | CA 2971419 A1 | 30 June 2016 |
| | | CA 2971419 C | 18 June 2024 |
| | | CN 107427573 A | 01 December 2017 |
| | | CN 107427573 B | 27 December 2022 |
| | | CN 116869964 A | 13 October 2023 |
| | | CN 116869964 B | 18 October 2024 |
| | | EP 3237003 A2 | 01 November 2017 |
| | | EP 3237003 A4 | 15 August 2018 |
| | | EP 3237003 B1 | 11 August 2021 |
| | | EP 3970748 A1 | 23 March 2022 |
| | | EP 3970748 B1 | 24 July 2024 |
| | | ES 2897707 T3 | 02 March 2022 |
| | | HK 1245661 A1 | 31 August 2018 |
| | | IL 253036 B | 30 September 2021 |
| | | IL 286025 A | 31 October 2021 |
| | | IL 286025 B1 | 01 July 2023 |
| | | IL 286025 B2 | 01 November 2023 |
| | | JP 2018-503625 A | 08 February 2018 |
| | | JP 2020-188789 A | 26 November 2020 |
| | | JP 2022-166133 A | 01 November 2022 |
| | | JP 6743022 B2 | 19 August 2020 |
| | | JP 7127876 B2 | 30 August 2022 |
| | | JP 7542870 B2 | 02 September 2024 |
| | | KR 10-2023-0144092 A | 13 October 2023 |
| | | KR 10-2582035 B1 | 22 September 2023 |
| | | MX 2017008487 A | 19 February 2018 |
| | | MX 2022003698 A | 26 April 2022 |
| | | SG 11201705064 XA | 28 July 2017 |
| | | US 10632193B 2 | 28 April 2020 |
| | | US 11510981B 2 | 29 November 2022 |
| | | US 2019-0022215 A1 | 24 January 2019 |
| | | US 2020-0297843 A1 | 24 September 2020 |
| | | US 2024-0066120 A1 | 29 February 2024 |
| | | WO 2016-105542 A2 | 30 June 2016 |
| | | WO 2016-105542 A3 | 27 October 2016 |
| KR 10-2020-0132147 A | 25 November 2020 | US 2022-0259562 A1 | 18 August 2022 |
| | | WO 2020-231205 A1 | 19 November 2020 |
| US 2013-0337528 A1 | 19 December 2013 | AU 2011-340132 A1 | 02 May 2013 |
| | | CA 2820861 A1 | 14 June 2012 |
| | | CN 103384677 A | 06 November 2013 |
| | | EP 2649088 A1 | 16 October 2013 |

Form PCT/ISA/210 (patent family annex) (July 2022)

**INTERNATIONAL SEARCH REPORT**
**Information on patent family members**

International application No.
**PCT/KR2024/017375**

| Patent document cited in search report | Publication date (day/month/year) | Patent family member(s) | Publication date (day/month/year) |
|---|---|---|---|
| | | EP 2649088 A4 | 03 September 2014 |
| | | JP 2014-506873 A | 20 March 2014 |
| | | KR 10-2014-0092227 A | 23 July 2014 |
| | | SG 191089 A1 | 31 July 2013 |
| | | WO 2012-077080 A1 | 14 June 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230152073
- KR 1020240021286
- KR 1020240154399